# EUROPÄISCHE PATENTSCHRIFT

(11) **EP 1 934 208 B1**
(45) Veröffentlichungstag und Bekanntmachung des Hinweises auf die Patenterteilung: **23.03.2011**
(21) Anmeldenummer: 06792210.4
(22) Anmeldetag: 22.09.2006
(51) Int. Cl.: C07D 413/14, A61K 31/5377, A61P 7/02

(54) **NEUE POLYMORPHE FORM VON 5-CHLOR-N-({ ( 5S )-2-0X0-3-[4-( 3-OXO-4-MORPHOLINYL)-PHENYL]-1,3-OXAZOLIDIN-5-YL} -METHYL)-2-THIOPHENCARBOXAMID**
NOVEL POLYMORPHOUS FORM OF 5-CHLORO-N-({ (5S)-2-OXO-3-[4-(3-OXO-4-MORPHOLINYL)-PHENYL]-1,3-OXAZOLIDINE-5-YL}-METHYL)-2-THIOPHENE CARBOXAMIDE
NOUVELLE FORME POLYMORPHE DE 5-CHLORO-N-({(5S)-2-OXO-3-[4-(3-OXO-4-MORPHOLINYL)-PHENYL]-1,3-OXAZOLIDIN-5-YL}-METHYL)-2-THIOPHENECARBOXAMIDE

(30) Priorität: 04.10.2005 DE 102005047564; 04.10.2005 DE 102005047563
(43) Veröffentlichungstag der Anmeldung: 25.06.2008
(60) Teilanmeldung: 10177691.2
(73) Patentinhaber: Bayer Schering Pharma Aktiengesellschaft, 13353 Berlin (DE)
(72) Erfinder: GRUNENBERG, Alfons, 41539 Dormagen (DE); LENZ, Jana, 70771 Leinfelden-Echterdingen (DE); BRAUN, Gerhard, Arnold, 50733 Köln (DE); KEIL, Birgit, 40231 Düsseldorf (DE); THOMAS, Christian, R., 42113 Wuppertal (DE)
(86) Internationale Anmeldenummer: PCT/EP2006/009202
(87) Internationale Veröffentlichungsnummer: WO 2007/039132

(56) Entgegenhaltungen:
- WO-A-2004/060887
- WO-A-2005/068456
- WO-A1-01/47919
- WO-A1-01/47949
- DE-A1- 10 129 725
- ROEHRIG, S. ET AL.: "Discovery of the Novel Antithrombotic Agent 5-Chloro-N-(((5S)-2-oxo-3-[4-(3-oxomorphol in-4-yl)phenyl]-1,3-oxazolidin-5-yl)methyl )thiophene-2-carboxamide (BAY 59-7939): An Oral, Direct Factor Xa Inhibitor" J. MED. CHEM., Bd. 48, 22. September 2005 (2005-09-22), Seiten 5900-5908, XP002418821
- CAIRA M R: "CRYSTALLINE POLYMORPHISM OF ORGANIC COMPOUNDS" TOPICS IN CURRENT CHEMISTRY, SPRINGER, BERLIN, DE, Bd. 198, 1998, Seiten 163-208, XP001156954 ISSN: 0340-1022
- HANCOCK B C ET AL: "CHARACTERISTICS AND SIGNIFICANCE OF THE AMORPHOUS STATE IN PHARMACEUTICAL SYSTEMS" JOURNAL OF PHARMACEUTICAL SCIENCES, AMERICAN PHARMACEUTICAL ASSOCIATION. WASHINGTON, US, Bd. 86, Nr. 1, Januar 1997 (1997-01), Seiten 1-12, XP000929450 ISSN: 0022-3549
- BERNSTEIN J.: 'Polymorphism in Molecular Crystals', 2002, CLARENDON PRESS, OXFORD * Zitiert als allgemeines Fachwissen.; page 104 - page 107 *
- BYRN S.R. ET AL: 'Solid-State Chemistry of Drugs, 2nd edition', 1999, SSCI, INC., WEST LAFAYETTE * Zitiert als allgemeines Fachwissen.; page 83 - page 85 *
- BRITTAIN H.G. (ED.): 'Polymorphism in Pharmaceutical Solids', 1999, MARCEL DEKKER, NEW YORK * Zitiert als allgemeines Fachwissen.; page 208 - page 217 *
- STOE & CIE GMBH: 'Broschüre STOE STADI MP', STOE & CIE GMBH, DARMSTADT pages 1 - 4 * Ohne Publikationsdatum. *
- Moore: "Physikalische Chemie, 3. Auflage" 1983, Walter de Gruyter , Berlin , page 1049
- Moore: "Physikalische Chemie, 3. Auflage" 1983, Walter de Gruyter , page 238
- Smith; Dent: "Modern Raman Spectroscopy - A Practical Approach" 2005, John Wiley & Sons, Ltd. , Chichester , pages 31-32
- "Europäisches Arzneibuch, 5. Ausgabe Grundwerk 2005" 2005, Deutscher Apotheker Verlag , Stuttgart vol. 1, , pages 99-101
- "Chapter 851" In: "The United States Pharmacopeia USP 32 NF 27" 1 May 2009 (2009-05-01), The United States Pharmacopeial Convention , Rockville vol. 1, * 10 Seiten. *
- "Fourierspekrometer IFS 28 - Benutzerhandbuch für Bedienung und Wartung (Vorläufige Version)" 1993, Bruker Analytische Messtechnik GmbH , page 14
- "Europäisches Arzneibuch, 5. Ausgabe Grundwerk 2005" 2005, Deutscher Apotheker Verlag , Stuttgart vol. 1, , pages 73-76
- STOE & CIE GMBH, DARMSTADT: 'STOE Stadi MP', [Online] pages 1 - 2 Retrieved from the Internet: <URL:www.stoe.com/pages/products/stadimp.ht ml> [retrieved on 2010-08-09] & STOE & CIE GMBH, DARMSTADT: 'STOE Stadi P combi', [Online] pages 1 - 2 Retrieved from the Internet: <URL:www.stoe.com/pages/products/stadipcomb i.html> [retrieved on 2010-08-09] & STOE & Cie GmbH, Darmsatdt: "STOE Stadi P" pages 1-2, Retrieved from the Internet: URL:www.stoe.com/pages/products/stadip.htm l [retrieved on 2010-08-09]
- STOE & Cie GmbH, Darmstadt: "Broschüre: STOE STADI P POWDER DIFFRACTOMETER SYSTEM" pages 1-4, Retrieved from the Internet: URL:http://www.stoe.com/pages/brochure/sta di_p_brochure.pdf [retrieved on 2010-08-09]
- Brittain, H. G. (ed.): "Polymorphism in Pharmaceutical Solids" 1999, Marcel Dekker , New York , pages 235-236 * Zitiert als allgemeines Fachwissen. *

## Beschreibung

Die vorliegende Erfindung betrifft eine neue polymorphe Form von 5-Chlor-N-({(5*S*)-2-oxo-3-[4-(3-oxo-4-morpholinyl)-phenyl]-1,3-oxazolidin-5-yl)-methyl)-2-thiophen-carboxamid, Verfahren zu deren Herstellung, diese enthaltende Arzneimittel sowie deren Verwendung bei der Bekämpfung von Krankheiten.

Die Verbindung 5-Chlor-N-({(5*S*)-2-oxo-3-[4-(3-oxo-4-morpholinyl)-phenyl]-1,3-oxazolidin-5-yl}-methyl)-2-thiophencarboxamid ist aus WO 01/47919 und WO 2004/060887 bekannt und entspricht der Formel (I):

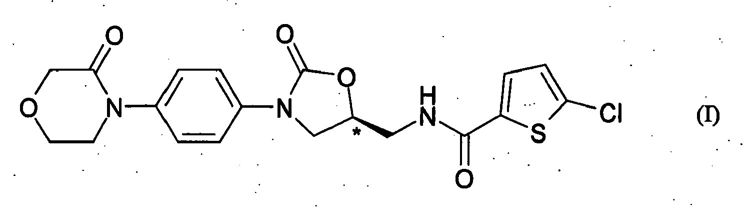

Die Verbindung der Formel (I) ist ein niedermolekularer, oral applizierbarer Inhibitor des Blutgerinnungsfaktors Xa, der zur Prophylaxe, Sekundärprophylaxe und/oder Behandlung von verschiedenen thromboembolischen Erkrankungen eingesetzt werden kann (siehe hierzu WO 01/47919, deren Offenbarung hiermit durch Bezugnahme eingeschlossen ist), insbesondere von Herzinfakt, Angina Pectoris (eingeschlossen instabile Angina), Reokklusionen und Restenosen nach einer Angioplastie oder aortokoronarem Bypass, Hirnschlag, transitorischen ischämischen Attacken, peripheren arteriellen Verschlusskrankheiten, Lungenembolien oder tiefen venösen Thrombosen.

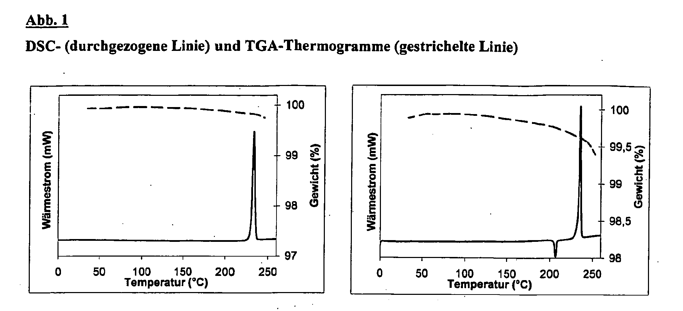

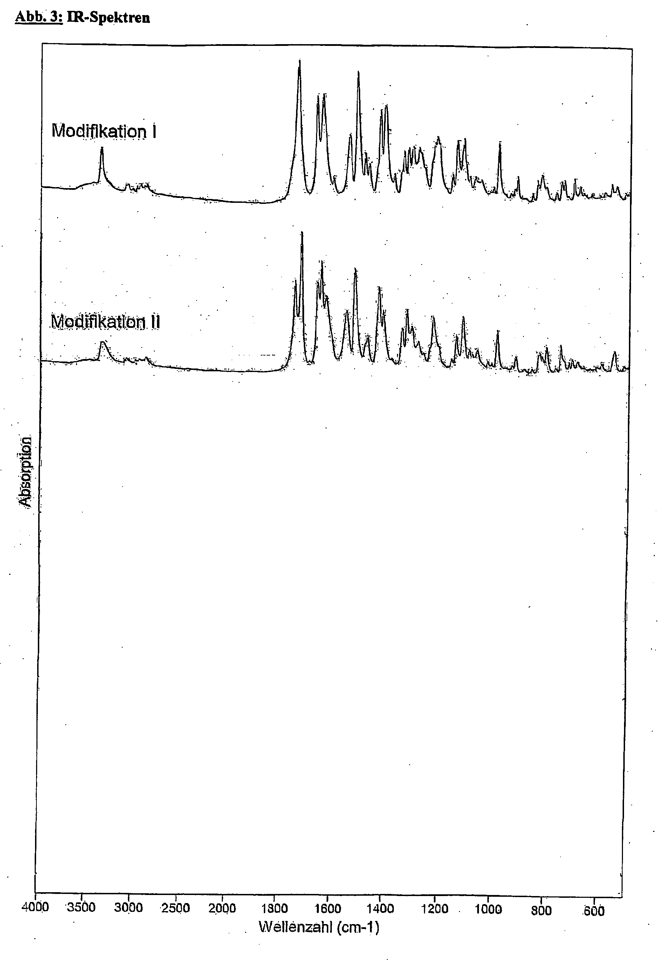

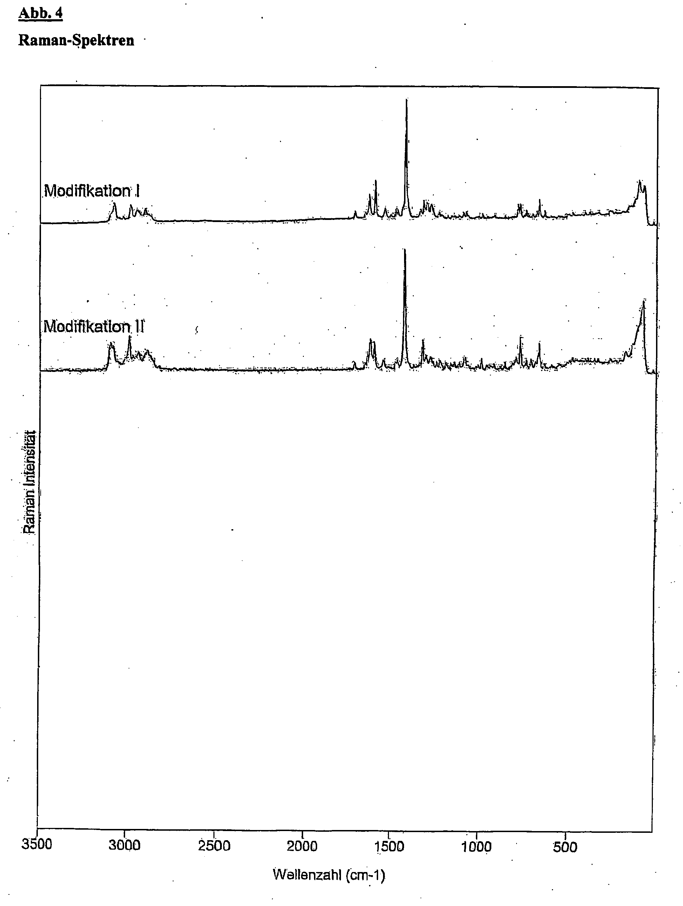

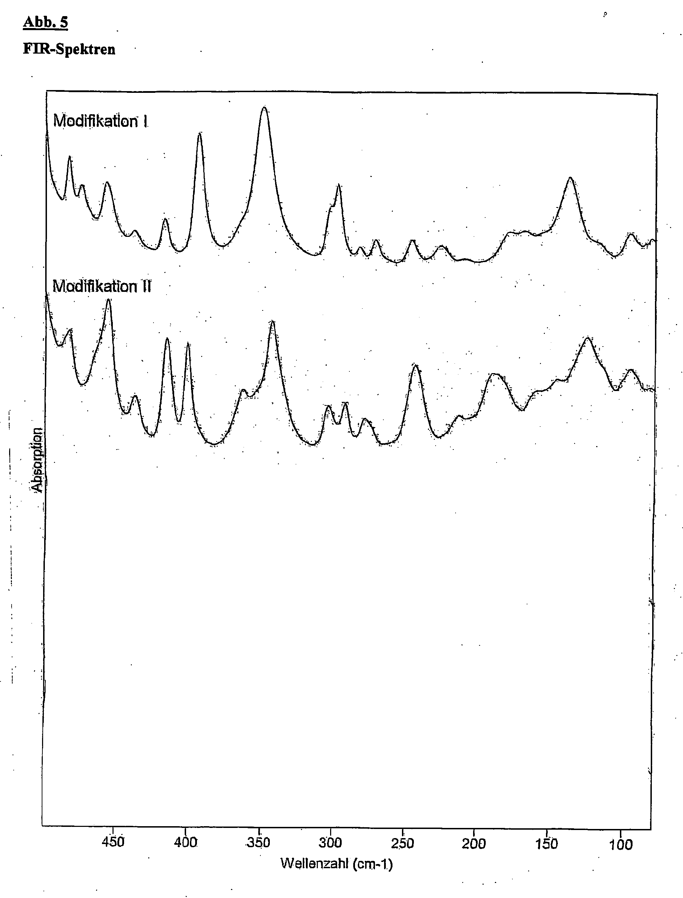

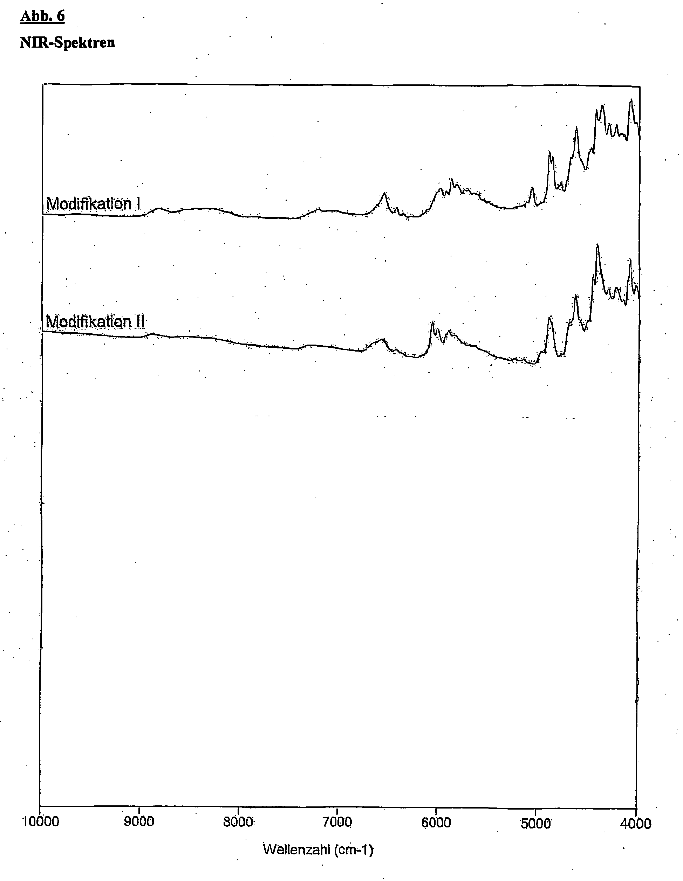

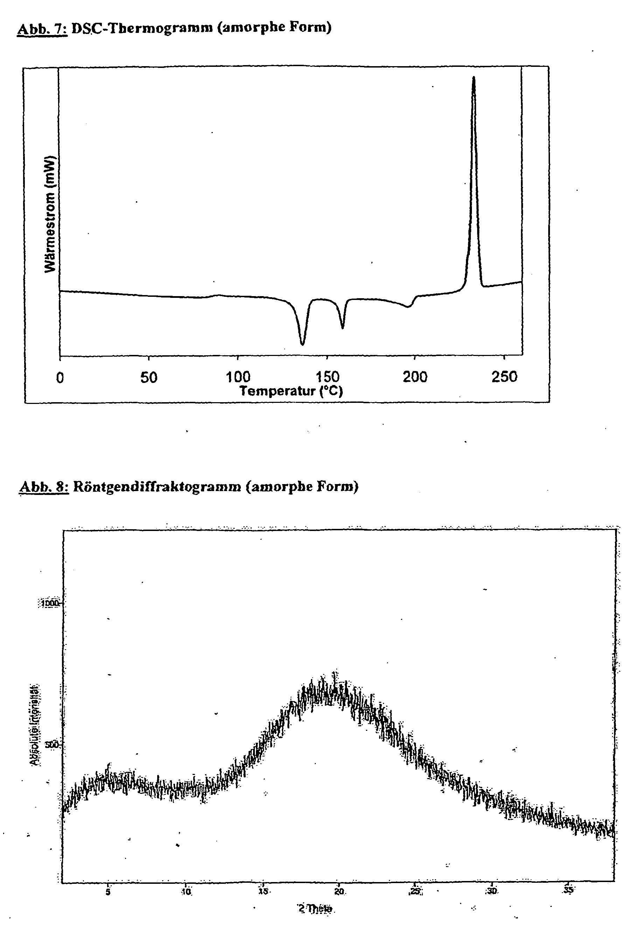

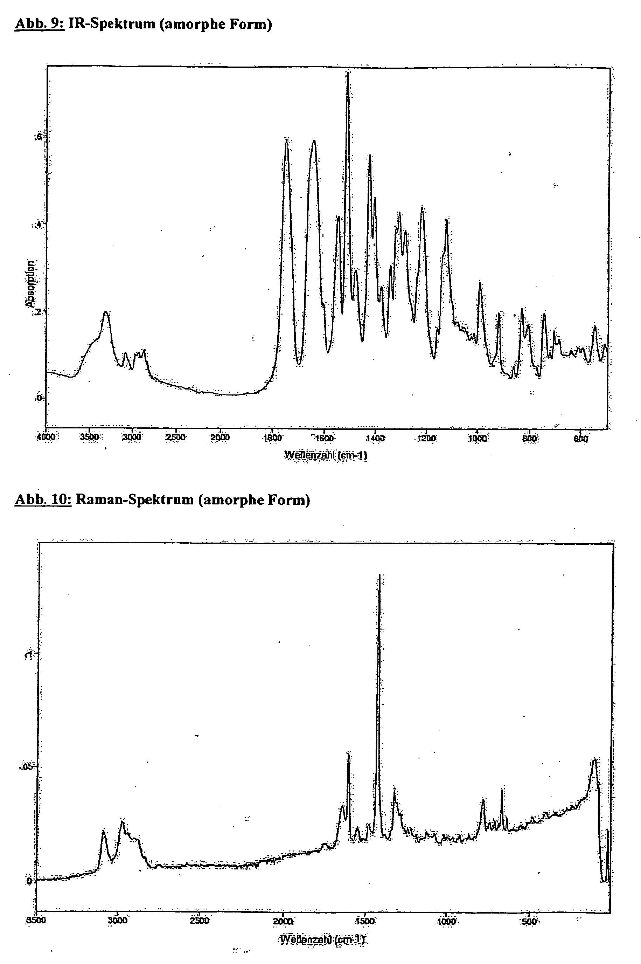

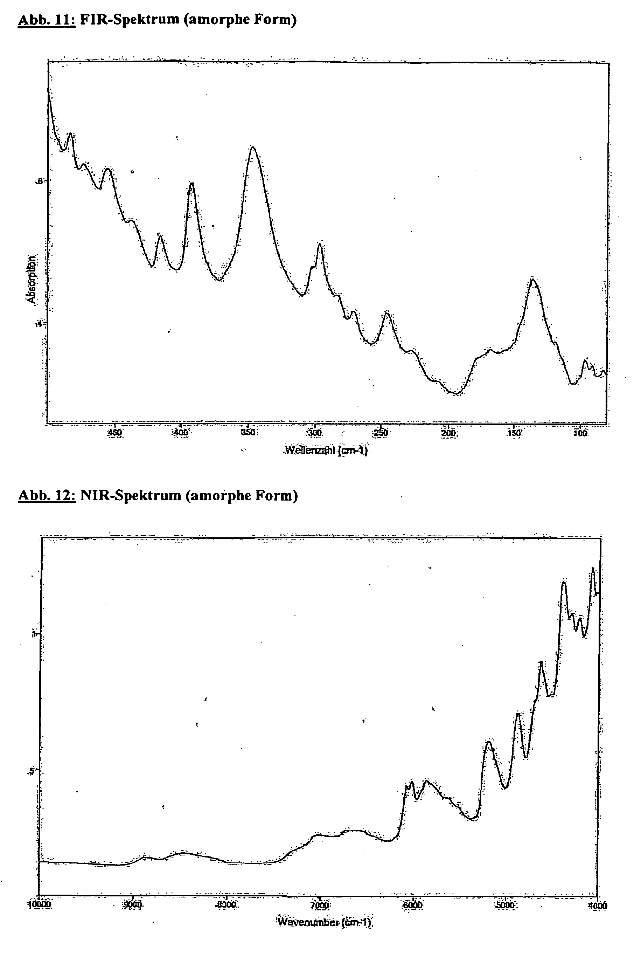

Die Verbindung der Formel (I) lässt sich wie in WO 01/47919 und WO 2004/060887 beschrieben herstellen. Dabei wird die Verbindung der Formel (I) in einer Kristallmodifikation erhalten, die im Folgenden als Modifikation I bezeichnet wird. Modifikation I hat einen Schmelzpunkt von 230°C und ein charakteristisches Röntgendiffraktogramm, IR-Spektrum, Raman-Spektrum, FIR-Spektrum und NIR-Spektrum (Tab. 1-6, Abb. 1-6). Es wurde nun gefunden, dass Modifikation I im Vergleich zur Modifikation II eine um den Faktor 4 geringere Löslichkeit aufweist.

Überraschenderweise wurden eine weitere Modifikation der Verbindung der Formel (I) gefunden. Die Verbindung der Formel (I) in der Modifikation II schmilzt bei etwa 203°C bzw. hat einen Umwandlungspunkt von etwa 195°C. Gegenstand der vorliegenden Erfindung ist die Verbindung der Formel (I) in der Modifikation II. Durch den erfindungsgemäßen Einsatz der Verbindung der Formel (I) in der Modifikation II wird sichergestellt, dass eine im Vergleich zur bekannten Modifikation höhere Löslichkeit erreicht wird.

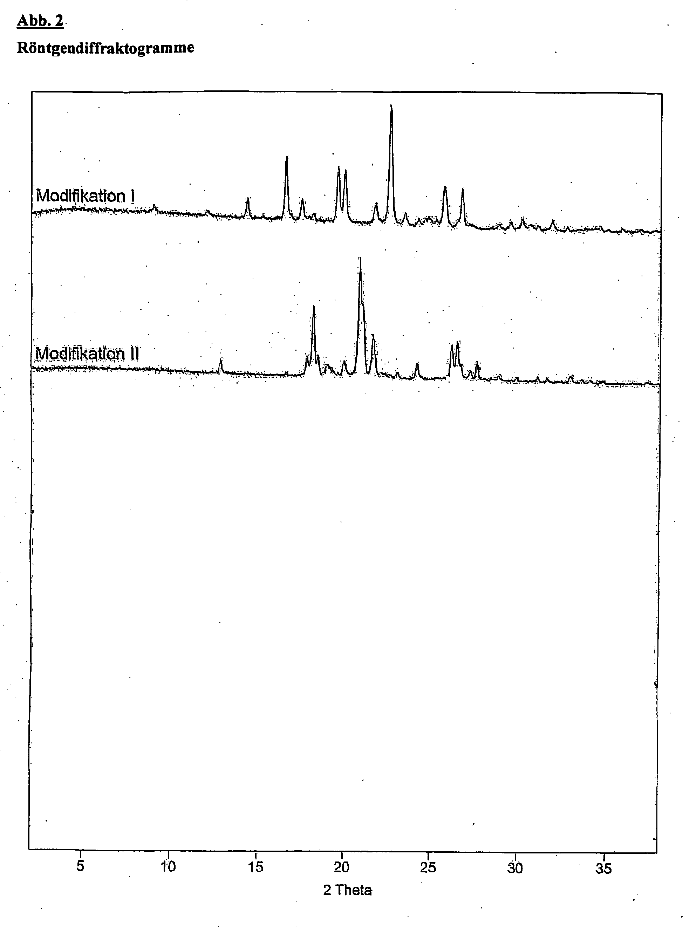

Modifikation II der Verbindung der Formel (I) hat im Vergleich zu Modifikation I, ein klar unterscheidbares Röntgendiffraktogramm, IR-Spektrum, NIR-Spektrum, FIR-Spektrum und Raman-Spektrum (Abb. 2-6). Die Verbindung der Formel (I) in der Modifikation II schmilzt bei 203°C bzw. wandelt sich bei etwa 195°C um und ist damit klar unterscheidbar von Modifikation I (Schmelzpunkt 230°C).

Die erfindungsgemäße Verbindung der Formel (I) in der Modifikation II wird in pharmazeutischen Formulierungen in hoher Reinheit eingesetzt. Aus Stabilitätsgründen enthält eine pharmazeutische Formulierung hauptsächlich die Verbindung der Formel (I) in der Modifikation II und keine größeren Anteile einer anderen Form wie beispielsweise einer anderen Modifikation oder eines Solvates der Verbindung der Formel (I). Bevorzugt enthält das Arzneimittel mehr als 90 Gewichtsprozente, besonders bevorzugt mehr als 95 Gewichtsprozente der Verbindung der Formel (I) in der Modifikation II bezogen auf die Gesamtmenge der enthaltenen Verbindung der Formel (I).

Weiterer Gegenstand der vorliegenden Erfindung ist der Einsatz der Verbindung der Formel (I) in der Modifikation II zur Behandlung und/oder Prophylaxe von Erkrankungen, vorzugsweise von thromboembolischen Erkrankungen und/oder thromboembolischen Komplikationen.

Zu den "thromboembolischen Erkrankungen" im Sinne der vorliegenden Erfindung zählen insbesondere Erkrankungen wie Herzinfarkt mit ST-Segment-Erhöhung (STEMI) und ohne ST-Segment-Erhöhung (non-STEMI), stabile Angina Pectoris, instabile Angina Pectoris, Reokklusionen und Restenosen nach Koronarinterventionen wie Angioplastie oder aortokoronarem Bypass, periphere arterielle Verschlusskrankheiten, Lungenembolien, tiefe venöse Thrombosen und Nierenvenenthrombosen, transitorische ischämische Attacken sowie thrombotischer und thromboembolischer Hirnschlag.

Die erfindungsgemäße Verbindung eignet sich daher auch zur Prävention und Behandlung von kardiogenen Thromboembolien, wie beispielsweise Hirn-Ischämien, Schlaganfall und systemischen Thromboembolien und Ischämien, bei Patienten mit akuten, intermittierenden oder persistierenden Herzarrhythmien, wie beispielsweise Vorhofflimmern, und solchen, die sich einer Kardioversion unterziehen, ferner bei Patienten mit Herzklappen-Erkrankungen oder mit künstlichen Herzklappen. Darüber hinaus ist die erfmdungsgemäße Verbindung zur Behandlung der disseminierten intravasalen Gerinnung (DIC) geeignet.

Thromboembolische Komplikationen treten ferner auf bei mikroangiopathischeri hämolytischen Anämien, extrakorporalen Blutkreisläufen, wie Hämodialyse, sowie Herzklappenprothesen.

Außerdem kommt die erfindungsgemäße Verbindung auch für die Prophylaxe und/oder Behandlung von atherosklerotischen Gefäßerkrankungen und entzündlichen Erkrankungen wie rheumatische Erkrankungen des Bewegungsapparats in Betracht, darüber hinaus ebenso für die Prophylaxe und/oder Behandlung der Alzheimer'schen Erkrankung. Außerdem kann die erfindungsgemäße Verbindung zur Inhibition des Tumorwachstums und der Metastasenbildung, bei Mikroangiopathien, altersbedingter Makula-Degeneration, diabetischer Retinopathie, diabetischer Nephropathie und anderen mikrovaskulären Erkrankungen sowie zur Prävention und Behandlung thromboembolischer Komplikationen, wie beispielsweise venöser Thromboembolien, bei Tumorpatienten, insbesondere solchen, die sich größeren chirurgischen Eingriffen oder einer Chemo- oder Radiotherapie unterziehen, eingesetzt werden.

Die erfindungsgemäße Verbindung kann darüber hinaus auch zur Verhinderung von Koagulation *ex vivo* eingesetzt werden, z.B. zur Konservierung von Blut- und Plasmaprodukten, zur Reinigung/Vorbehandlung von Kathetern und anderen medizinischen Hilfsmitteln und Geräten, zur Beschichtung künstlicher Oberflächen von *in vivo* oder *ex vivo* eingesetzten medizinischen Hilfsmitteln und Geräten oder bei biologischen Proben, die Faktor Xa enthalten.

Weiterer Gegenstand der vorliegenden Erfindung ist die Verwendung der erfindungsgemäßen Verbindung zur Behandlung und/oder Prophylaxe von Erkrankungen, insbesondere der zuvor genannten Erkrankungen.

Weiterer Gegenstand der vorliegenden Erfindung ist die Verwendung der erfindungsgemäßen Verbindung zur Herstellung eines Arzneimittels zur Behandlung und/oder Prophylaxe von Erkrankungen, insbesondere der zuvor genannten Erkrankungen.

Weiterer Gegenstand der vorliegenden Erfindung ist ein Verfahren zur Behandlung und/oder Prophylaxe von Erkrankungen, insbesondere der zuvor genannten Erkrankungen, unter Verwendung einer antikoagulatorisch wirksamen Menge der erfindungsgemäßen Verbindung.

Weiterer Gegenstand der vorliegenden Erfindung ist ein Verfahren zur Verhinderung der Blutkoagulation *in vitro,* insbesondere bei Blutkonserven oder biologischen Proben, die Faktor Xa enthalten, das dadurch gekennzeichnet ist, dass eine antikoagulatorisch wirksame Menge der erfindungsgemäßen Verbindung zugegeben wird.

Weiterer Gegenstand der vorliegenden Erfindung sind Arzneimittel, enthaltend die erfindungsgemäße Verbindung und einen oder mehrere weitere Wirkstoffe, insbesondere zur Behandlung und/oder Prophylaxe der zuvor genannten Erkrankungen. Als geeignete Kombinationswirkstoffe seien beispielhaft und vorzugsweise genannt:
- Lipidsenker, insbesondere HMG-CoA-(3-Hydroxy-3-methylglutaryl-Coenzym A)-Reduktase-Inhibitoren;
- Koronartherapeutika/Vasodilatatoren, insbesondere ACE-(Angiotensin-Converting-Enzyme)-Inhibitoren; AII-(Angiotensin II)-Rezeptor-Antagonisten; β-Adrenozeptor-Antagonisten; alpha-1-Adrenozeptor-Antagonisten; Diuretika; Calciumkanal-Blocker; Substanzen, die eine Erhöhung von cyclischem Guanosinmonophosphat (cGMP) bewirken, wie beispielsweise Stimulatoren der löslichen Guanylatcyclase;
- Plasminogen-Aktivatoren (Thrombolytika/Fibrinolytika) und die Thrombolyse/Fibrinolyse steigernde Verbindungen wie Inhibitoren des Plasminogen-Aktivator-Inhibitors (PAI-Inhibitoren) oder Inhibitoren des Thrombin-aktivierten Fibrinolyse-Inhibitors (TAFI-Inhibitoren);
- antikoagulatorisch wirksame Substanzen (Antikoagulantien);
- plättchenaggregationshemmende Substanzen (Plättchenaggregationshemmer, Thrombozytenaggregationshemmer);
- sowie Fibrinogen-Rezeptor-Antagonisten (Glycoprotein-IIb/IIIa-Antagonisten).

Weiterer Gegenstand der vorliegenden Erfindung sind Arzneimittel, die die erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäße Verbindung kann systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege kann die erfindungsgemäße Verbindung in geeigneten Applikationsformen verabreicht werden.

Für die orale Applikation eignen sich nach dem Stand der Technik funktionierende, die erfindungsgemäß Verbindung schnell und/oder modifiziert abgebende Applikationsformen, die die Verbindung der Formel (I) in der Modifikation II oder in der amorphen Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Suspensionen oder Aerosole.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Suspensionen, Lyophilisaten oder sterilen Pulvern.

Für die sonstigen Applikationswege eignen sich z.B. Inhalationsarzneiformen (u.a. Pulverinhalatoren, Nebulizer), lingual, sublingual oder buccal zu applizierende Tabletten, Filme/Oblaten oder Kapseln, Suppositorien, Ohren- oder Augenpräparationen, Vaginalkapseln, wässrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale Applikation.

Die erfindungsgemäße Verbindung kann in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Weiterer Gegenstand der Erfindung ist ein Verfahren zur Herstellung der Verbindung der Formel (I) in der Modifikation II, indem die Verbindung der Formel (I) in der Modifikation I in einem inerten Lösungsmittel gelöst und der Wirkstoff durch Zugabe eines Fällungsmittels bei einer Temperatur zwischen 0°C und 80°C, bevorzugt von 20 bis 25°C, gefällt wird. Der Niederschlag wird isoliert und getrocknet. Man erhält so die Verbindung der Formel (I) in der Modifikation II.

Ebenfalls Gegenstand der Erfindung ist ein Verfahren zur Herstellung der Verbindung der Formel (I) in der Modifikation II, indem die Verbindung der Formel (I) in der Modifikation I in einem inerten Lösungsmittel gelöst und, bevorzugt bei erhöhter Temperatur, insbesondere bei einer Temperatur von 30°C bis zur Rückflusstemperatur des Lösungsmittels, bis zum vollständigen Verdampfen des Lösungsmittels und Auskristallisieren des Wirkstoffs gelagert wird. Man erhält so die Verbindung der Formel (I) in der Modifikation II.

Ebenfalls Gegenstand der Erfindung ist ein Verfahren zur Herstellung der Verbindung der Formel (I) in der Modifikation II, indem die Verbindung der Formel (I) in der amorphen Form in einem wasserfreien inerten Lösungsmittel suspendiert und bis zum Erreichen des gewünschten Umwandlungsgrads, insbesondere bis zur quantitativen Umwandlung, in die Modifikation n gerührt oder geschüttelt wird. Das erhaltene Kristallisat wird isoliert und getrocknet. Man erhält so die Verbindung der Formel (I) in der Modifikation II.

Als inerte Lösungsmittel eignen sich niedere Alkohole wie beispielsweise Methanol, Ethanol, n-Propanol, iso-Propanol, n-Butanol, sec-Butanol, iso-Butanol, 1-Pentanol, oder Ketone wie Aceton, oder Alkane wie n-Pentan, Cyclopentan, n-Hexan, Cyclohexan, oder Tetrahydrofuran, oder Acetonitril, oder Toluol, oder Ethylacetat, oder 1,4-Dioxan, oder Gemische der genannten Lösungsmittel, oder Gemische der genannten Lösungsmittel mit Wasser, Bevorzugt sind Aceton, Tetrahydrofuran, 1-Pentanol oder Gemische der genannten Lösungsmittel. Als Fällungsmittel eignen sich inerte, wasserfreie Lösungsmittel, in denen der Wirkstoff schwer löslich ist, wie z. B. n-Heptan, Cyclohexan oder Toluol. Bevorzugt ist n-Heptan.

Bevorzugt wird die Verbindung der Formel (I) in der Modifikation II hergestellt, indem die Verbindung der Formel (I) in der Modifikation I in Aceton oder Tetrahydrofuran gelöst und der Wirkstoff durch Zugabe von n-Heptan bei einer Temperatur zwischen 0 und 80°C, bevorzugt bei einer Temperatur von 20 bis 25°C, gefällt wird. Der Niederschlag wird isoliert und getrocknet. Man erhält so die Verbindung der Formel (I) in der Modifikation II.

Ebenfalls bevorzugt wird die Verbindung der Formel (I) in der Modifikation II hergestellt, indem die Verbindung der Formel (I) in der Modifikation I in 1,4-Dioxan gelöst wird und bei erhöhter Temperatur, insbesondere bei einer Temperatur von 30°C bis zur Rückflusstemperatur des Lösungsmittels, beispielsweise 50°C, bis zum vollständigen Verdampfen des Lösungsmittels und Auskristallisieren des Wirkstoffs gelagert wird. Man erhält so die Verbindung der Formel (I) in der Modifikation II.

Ebenfalls bevorzugt wird die Verbindung der Formel (I) in der Modifikation II hergestellt, indem die Verbindung der Formel (I) in der amorphen Form in einem inerten wasserfreien Lösungsmittel suspendiert und bis zum Erreichen des gewünschten Umwandlungsgrads in die Modifikation II bei einer Temperatur von 20 bis 25°C gerührt oder geschüttelt wird. Das erhaltene Kristallisat wird isoliert und getrocknet Man erhält so die Verbindung der Formel (I) in der Modifikation II.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### Ausführungsbeispiele

Die Thermogramme wurden unter Verwendung eines Differential Scanning Calorimeters DSC 7 bzw. Pyris-1 und Thermogravimetric Analyser TGA 7 der Fa. Perkin-Elmer erhalten. Die Röntgendiffraktogramme wurden in einem Stoe-Transmissionsdiffraktometer registriert. Die IR-, FIR-, NIR- und Raman-Spektren wurden mit Fourier-IR-Spektrometern IFS 66v (IR, FIR), IFS 28/N (NIR) und RFS 100 (Raman) der Fa. Bruker aufgenommen.

### Beispiel 1: 5-Chlor-N-({(5S)-2-oxo-3-[4-(3-oxo-4-morpholinyl)-phenyl]-1,3-oxazolidin-5-yl}-methyl)-2-thiophencarboxamid in der Modifikation I

Die Herstellung der Modifikation I der Titelverbindung ist in WO 01/47919 und WO 2004/060887 beschrieben.

### Beispiel 2: Herstellung von 5-Chlor-N-({(5S)-2-oxo-3-[4-(3-oxo-4-morpholinyl)-phenyl]1-1,3-oxazolidin-5-yl}-methyl)-2-thiophencarboxamid in der Modifikation II

### Beispiel 2.1

208 g Chlorthiophencarbonsäure wurden in 1100 ml Toluol suspendiert und auf 75 bis 80°C erhitzt. Bei dieser Temperatur wurden innerhalb von 2 h 112 ml Thionylchlorid zugetropft. Die entstandene Reaktionslösung wurde bis zum Ende der Gasentwicklung weitere 2 h nachgerührt. Dabei wurde die Innentemperatur in 5°-Schritten auf 100-110°C erhöht. Das Gemisch wurde abgekühlt und die Lösung des Säurechlorids am Rotationsverdampfer eingeengt.

350 g Oxamin-Hydrochlorid wurden in 2450 ml NMP suspendiert, mit 385 ml Triethylamin versetzt und 15 min gerührt. Das Gemisch wurde auf 10°C gekühlt, mit der Lösung aus dem Säurechlorid und 70 ml Toluol versetzt und gerührt. Zu der Suspension wurden 350 ml Leitungswasser gegeben und auf 82°C erhitzt. Nach Filtration wurde der Wirkstoff mit 3,5 1 Wasser gefällt und 2 h nachgerührt. Trocknung bei 70°C im Vakuum.

### Beispiel 2.2

Ca. 200 mg 5-Chlor-N-({(5*S*)-2-oxo-3-[4-(3-oxo-4-morpholinyl)-phenyl]-1,3-oxazolidin-5-yl}-methyl)-2-thiophencarboxamid in der Modifikation I wurden in ca. 80 ml Tetrahydrofuran heiß gelöst. Die Lösung wurde filtriert und halbiert. Eine Hälfte wurde bei Raumtemperatur mit n-Heptan versetzt, bis der Wirkstoffausfiel. Der Rückstand wurde abfiltriert und bei Raumtemperatur getrocknet. Er wurde röntgendiffraktometrisch untersucht und entsprach der Titelverbindung in der Modifikation II.

### Beispiel 2.3

Ca. 200 mg 5-Chlor-N-({(5*S*)-2-oxo-3-[4-(3-oxo-4-morpholinyl)-phenyl]-1,3-oxazolidin-5-yl}-methyl)-2-thiophencarboxamid in der Modifikation I wurden in ca. 40 ml 1-Pentanol heiß gelöst. Die Lösung wurde filtriert und halbiert. Ein Hälfte wurde mit n-Heptan versetzt, bis der Wirkstoffausfiel. Der Rückstand wurde abfiltriert und bei Raumtemperatur getrocknet. Er wurde röntgendiffraktometrisch untersucht und entsprach der Titelverbindung in der Modifikation II.

### Beispiel 2.4

Ca. 200 mg 5-Chlor-N-({(5*S*)-2-oxo-3-[4-(3-oxo-4-morpholinyl)-phenyl]-1,3-oxazolidin-5-yl}-methyl)-2-thiophencarboxamid in der Modifikation I wurden in ca. 40 ml 1,4-Dioxan heiß gelöst. Die Lösung wurde filtriert und halbiert. Eine Hälfte wurde bei 50°C im Trockenschrank gelagert, bis das Lösungsmittel verdampft war. Der Rückstand wurde röntgendiffraktometrisch untersucht und entsprach der Titelverbindung in der Modifikation II.

### Beispiel 2.5

Ca. 50 mg 5-Chlor-N-({(5*S*)-2-oxo-3-[4-(3-oxo-4-morpholinyl)-phenyl]-1,3-oxazolidin-5-yl}-methyl)-2-thiophencarboxamid in der amorphen Form, hergestellt durch Durchschmelzen auf der Kofler-Heizbank bei ca. 240°C und anschließende Schockkühlung auf Raumtemperatur, wurden in ca. 2 ml Ethanol suspendiert und 0,5 h bei 25°C gerührt. Das Kristallisat wurde isoliert und getrocknet. Der Rückstand wurde röntgendiffraktometrisch untersucht und entsprach der Titelverbindung in der Modifikation II.

### Beispiel 2.6

Ca. 100 mg 5-Chlor-N-({(5*S*)-2-oxo-3-[4-(3-oxo-4-morpholinyl)-phenyl]-1,3-oxazolidih-5-yl}-methyl)-2-thiophencarboxamid in der Modifikation I wurden in ca. 50 ml Aceton heiß gelöst. Die Lösung wurde filtriert und im Eisbad mit n-Heptan versetzt, bis der Wirkstoff ausfiel. Der Rückstand wurde abfiltriert und bei Raumtemperatur getrocknet. Er wurde röntgendiffraktometrisch untersucht und entsprach der Titelverbindung in der Modifikation II.

Tab. 1: Differential Scanning Calorimetry und Thermogravimetrie

| | Modifikation I | Modifikation II |
|---|---|---|
| Schmelzpunkt [°C] | 230 | 203 |
| Umwandlungspunkt [°C] | - | ca. 192 |
| Masseverlust [Gew.-%] | 0,1 | 0,1 |

Tab. 2: Röntgendiffraktometrie

| Reflexe | |
|---|---|
| Modifikation I [2 Theta] | Modifikation II [2 Theta] |
| 8,9 | 12,8 |
| 12,0 | 17,7 |
| 14,3 | 18,1 |
| 16,5 | 18,4 |
| 17,4 | 19,0 |
| 18,1 | 19,9 |
| 19,5 | 20,8 |
| 19,9 | 21,6 |
| 21,7 | 22,1 |
| 22,5 | 22,9 |
| 23,4 | 24,1 |
| 24,1 | 26,1 |
| 24,5 | 26,4 |
| 24,7 | 26,6 |
| 25,6 | 27,2 |
| 26,4 | 27,5 |
| 26,7 | 28,8 |
| 30,0 | 29,8 |
| 30,1 | 31,0 |
| 31,8 | 31,6 |
| | 32,9 |

Tab. 3: IR-Spektroskopie

| Peakmaxima | |
|---|---|
| Modifikation I [cm⁻¹] | Modifikation II [cm⁻¹] |
| 564 | 552 |
| 686 | 598 |
| 708 | 692 |
| 746 | 713 |
| 757 | 725 |
| 830 | 756 |
| 846 | 809 |
| 920 | 825 |
| 991 | 833 |
| 1011 | 924 |
| 1056 | 994 |
| 1077 | 1067 |
| 1120 | 1085 |
| 1146 | 1097 |
| 1163 | 1121 |
| 1219 | 1146 |
| 1286 | 1232 |
| 1307 | 1285 |
| 1323 | 1310 |
| 1341 | 1328 |
| 1374 | 1345 |
| 1411 | 1415 |
| 1429 | 1431 |
| 1470 | 1473 |
| 1486 | 1523 |
| 1517 | 1554 |
| 1546 | 1631 |
| 1605 | 1648 |
| 1646 | 1663 |
| 1669 | 1723 |
| 1737 | 1745 |
| 2867 | 3341 |
| 2895 | |
| 2936 | |
| 2976 | |
| 3354 | |
| 1325 | 995 |
| 1343 | 1086 |
| 1428 | 1119 |
| 1473 | 1149 |
| 1485 | 1196 |
| 1548 | 1227 |
| 1605 | 1248 |
| 1638 | 1282 |
| 1664 | 1310 |
| 1722 | 1330 |
| 2899 | 1432 |
| 2944 | 1474 |
| 2983 | 1556 |
| 3074 | 1608 |
| | 1631 |
| | 1648 |
| | 1722 |
| | 2885 |
| | 2938 |
| | 2989 |
| | 3077 |
| | 3091 |

Tab. 5: FIR-Spektroskopie

| Peakmaxima | |
|---|---|
| Modifikation I [cm⁻¹] | Modifikation II [cm⁻¹] |
| 82 | 83 |
| 97 | 96 |
| 138 | 126 |
| 169 | 146 |
| 179 | 159 |
| 210 | 190 |
| 226 | 213 |
| 247 | 244 |
| 272 | 279 |
| 283 | 293 |
| 298 | 304 |
| 303 | 344 |
| 350 | 363 |
| 394 | 401 |
| 417 | 416 |
| 438 | 437 |
| 458 | 456 |
| 475 | 484 |
| 484 | |

Tab. 6: NIR-Spektroskopie

| Peakmaxima | |
|---|---|
| Modifiation I [cm⁻¹] | Modifikation II [cm⁻¹] |
| 4082 | 4086 |
| 4142 | 4228 |
| 4170 | 4418 |
| 4228 | 4457 |
| 4299 | 4634 |
| 4376 | 4905 |
| 4429 | 5846 |
| 4479 | 5911 |
| 4633 | 6026 |
| 4791 | 6081 |
| 4877 | 6582 |
| 4907 | |
| 5081 | |
| 5760 | |
| 5885 | |
| 6002 | |
| 6441 | |
| 6564 | |
| 8473 | |
| 8833 | |

### Beispiel 3 :Herstellung von 5-Chlor-N-({(5S)-2-oxo-3-[4-(3-oxo-4-morpholinyl)-phenyl]-1,3-oxazolidin-5-yl}-methyl)-2-thiophencarboxamid in amorpher Form

### Beispiel 3.1

Ca. 50 mg 5-Chlor-N-({(5*S*)-2-oxo-3-[4-(3-oxo-4-morpholinyl)-phenyl]-1,3-oxazolidin-5-yl}-methyl)-2-thiophencarboxamid in der Modifikation I wurden auf der Kofler-Heizbank bei ca. 240°C durchgeschmolzen und anschließend durch Schockkühlung auf Raumtemperatur gebracht. Der Wirkstoff wurde röntgendiffraktometrisch untersucht und lag in der amorphen Form vor.

### Beispiel 3.2

Ca. 3 g 5-Chlor-N-({(5*S*)-2-oxo-3-[4-(3-oxo-4-morpholinyl)-phenyl]-1,3-oxazolidin-5-yl}-methyl)-2-thiophencarboxamid in der Modifikation I wurden im Trockenschrank bei ca. 250°C durchgeschmolzen und anschließend durch Schockkühlung auf Raumtemperatur gebracht. Der Wirkstoff wurde röntgendiffraktometrisch untersucht und lag in der amorphen Form vor.

### Tab. 7: Differential Scanning Calorimetry und Thermogravimetrie (amorphe Form)

Glasumwandlungstemperatur: ca. 83°C

**Tab. 8: Spektroskopie (amorphe Form)**

| Peakmaxima | | | |
|---|---|---|---|
| IR [cm⁻¹] | Raman [cm⁻¹] | FIR [cm⁻¹] | NIR [cm⁻¹] |
| 467 | 486 | 91 | 4006 |
| 512 | 642 | 97 | 4081 |
| 550 | 673 | 137 | 4224 |
| 595 | 711 | 169 | 4307 |
| 613 | 742 | 246 | 4403 |
| 643 | 781 | 272 | 4634 |
| 689 | 923 | 297 | 4875 |
| 709 | 965 | 248 | 5193 |
| 725 | 1016 | 393 | 5865 |
| 750 | 1078 | 416 | 6017 |
| 810 | 1126 | 438 | 6073 |
| 834 | 1224 | 456 | 6696 |
| 864 | 1243 | 474 | 7028 |
| 921 | 1290 | 474 | 8452 |
| 995 | 1326 | | 8873 |
| 1015 | 1428 | | |
| 1026 | 1479 | | |
| 1058 | 1548 | | |
| 1083 | 1607 | | |
| 1126 | 1642 | | |
| 1161 | 2158 | | |
| 1222 | 2975 | | |
| 1288 | 3090 | | |
| 1312 | | | |
| 1325 | | | |
| 1380 | | | |
| 1407 | | | |
| 1428 | | | |
| 1480 | | | |
| 1516 | | | |
| 1549 | | | |
| 1607 | | | |
| 1647 | | | |
| 1753 | | | |
| 2126 | | | |
| 2869 | | | |
| 2933 | | | |
| 2967 | | | |
| 3084 | | | |
| 3317 | | | |

## Patentansprüche

1. Verbindung der Formel (I) 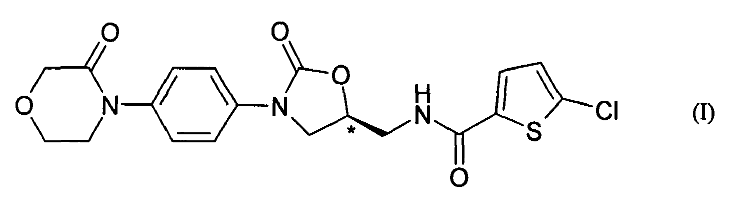 in der Modifikation II, die im NIR-Spektrogramm Peakmaxima bei 4086, 4228, 4418, 4457, 4634, 4905, 5846, 5911, 6026, 6081 und 6582 aufweist.

2. Verfahren zur Herstellung der Verbindung der Formel (I) gemäß Anspruch 1 in der Modifikation II, die im NIR-Spektrogramm Peakmaxima bei 4086, 4228, 4418, 4457, 4634, 4905, 5846, 5911, 6026, 6081 und 6582 aufweist, **dadurch gekennzeichnet, dass** die Verbindung der Formel (I) gemäß Anspruch 1 in der Modifikation I, die im NIR-Spektrogramm Peakmaxima bei 4082, 4142, 4170, 4228, 4299, 4376, 4429, 4479, 4633, 4791, 4877, 4907, 5081, 5760, 5885, 6002, 6441, 6564, 8473 und 8833 aufweist, in Methanol, Ethanol, n-Propanol, iso-Propanol, n-Butanol, sec-Butanol, iso-Butanol, 1-Pentanol, oder Aceton, oder n-Pentan, Cyclopentan, n-Hexan oder Tetrahydrofuran, oder Acetonitril, oder Ethylacetat, oder 1,4-Dioxan, oder einem Gemisch der genannten Lösungsmittel, oder einem Gemisch der genannten Lösungsmittel mit Wasser gelöst und die Verbindung durch Zugabe von n-Heptan, Cyclohexan oder Toluol gefällt wird.

3. Verfahren zur Herstellung der Verbindung der Formel (I) gemäß Anspruch 1 in der Modifikation II, die im NIR-Spektrogramm Peakmaxima bei 4086, 4228, 4418, 4457, 4634, 4905, 5846, 5911, 6026, 6081 und 6582 aufweist, **dadurch gekennzeichnet, dass** die Verbindung der Formel (I) gemäß Anspruch 1 in der amorphen Form in n-Heptan, Cyclohexan oder Toluol suspendiert und die Suspension bis zur quantitativen Umwandlung in die Modifikation II gerührt oder geschüttelt wird.

4. Verbindung der Formel (I) gemäß Anspruch 1 in der Modifikation II, die im NIR-Spektrogramm Peakmaxima bei 4086, 4228, 4418, 4457, 4634, 4905, 5846, 5911, 6026, 6081 und 6582 aufweist, zur Behandlung und/oder Prophylaxe von Krankheiten.

5. Verwendung der Verbindung der Formel (I) gemäß Anspruch 1 in der Modifikation II, die im NIR-Spektrogramm Peakmaxima bei 4086, 4228, 4418, 4457, 4634, 4905, 5846, 5911, 6026, 6081 und 6582 aufweist, zur Herstellung eines Arzneimittels zur Behandlung und/oder Prophylaxe von thromboembolischen Erkrankungen.

6. Verwendung der Verbindung der Formel (I) gemäß Anspruch 1 in der Modifikation II, die im NIR-Spektrogramm Peakmaxima bei 4086, 4228, 4418, 4457, 4634, 4905, 5846, 5911, 6026, 6081 und 6582 aufweist, zur Verhinderung der Blutkoagulation in vitro.

7. Arzneimittel enthaltend die Verbindung der Formel (I) gemäß Anspruch 1 in der Modifikation II, die im NIR-Spektrogramm Peakmaxima bei 4086, 4228, 4418, 4457, 4634, 4905, 5846, 5911, 6026, 6081 und 6582 aufweist, in Kombination mit einem inerten, nicht-toxischen, pharmazeutisch geeigneten Hilfsstoff.

8. Arzneimittel enthaltend die Verbindung der Formel (I) gemäß Anspruch 1 in der Modifikation II, die im NIR-Spektrogramm Peakmaxima bei 4086, 4228, 4418, 4457, 4634, 4905, 5846, 5911, 6026, 6081 und 6582 aufweist, in Kombination mit einem weiteren Wirkstoff.

9. Arzneimittel nach Anspruch 7 oder 8 zur Behandlung und/oder Prophylaxe von thromboembolischen Erkrankungen.

10. Verfahren zur Verhinderung der Blutkoagulation in vitro, **dadurch gekennzeichnet, dass** eine antikoagulatorisch wirksame Menge der Verbindung der Formel (I) gemäß Anspruch 1 in der Modifikation II, die im NIR-Spektrogramm Peakmaxima bei 4086, 4228, 4418, 4457, 4634, 4905, 5846, 5911, 6026, 6081 und 6582 aufweist, zugegeben wird.

## Claims

1. Compound of the formula (I) 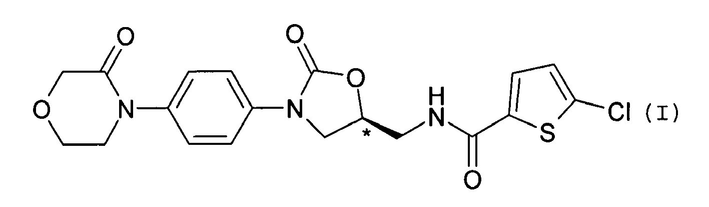 in the modification II which in the NIR spectrogram has peak maxima at 4086, 4228, 4418, 4457, 4634, 4905, 5846, 5911, 6026, 6081 and 6582.

2. Process for the preparation of the compound of the formula (I) according to Claim 1 in the modification II which in the NIR spectrogram has peak maxima at 4086, 4228, 4418, 4457, 4634, 4905, 5846, 5911, 6026, 6081 and 6582, **characterized in that** the compound of the formula (I) according to Claim 1 in the modification I which in the NIR spectrogram has peak maxima at 4082, 4142, 4170, 4228, 4299, 4376, 4429, 4479, 4633, 4791, 4877, 4907, 5081, 5760, 5885, 6002, 6441, 6564, 8473 and 8833, is dissolved in methanol, ethanol, n-propanol, isopropanol, n-butanol, sec-butanol, isobutanol, 1-pentanol, or acetone, or n-pentane, cyclopentane, n-hexane or tetrahydrofuran, or
acetonitrile, or ethyl acetate, or 1,4-dioxane, or a mixture thereof, or a mixture thereof with water and the compound is precipitated by addition of n-heptane, cyclohexane or toluene.

3. Process for the preparation of the compound of the formula (I) according to Claim 1 in the modification II which in the NIR spectrogram has peak maxima at 4086, 4228, 4418, 4457, 4634, 4905, 5846, 5911, 6026, 6081 and 6582, **characterized in that** the compound of the formula (I) according to Claim 1 in the amorphous form is suspended in n-heptane, cyclohexane or toluene and the suspension is stirred or shaken until quantitative conversion to the modification II.

4. Compound of the formula (I) according to Claim 1 in the modification II which in the NIR spectrogram has peak maxima at 4086, 4228, 4418, 4457, 4634, 4905, 5846, 5911, 6026, 6081 and 6582 for the treatment and/or prophylaxis of diseases.

5. Use of the compound of the formula (I) according to Claim 1 in the modification II which in the NIR spectrogram has peak maxima at 4086, 4228, 4418, 4457, 4634, 4905, 5846, 5911, 6026, 6081 and 6582 for the production of a medicament for the treatment and/or prophylaxis of thromboembolic diseases.

6. Use of the compound of the formula (I) according to Claim 1 in the modification II which in the NIR spectrogram has peak maxima at 4086, 4228, 4418, 4457, 4634, 4905, 5846, 5911, 6026, 6081 and 6582 for the prevention of blood coagulation in vitro.

7. Medicament comprising the compound of the formula (I) according to Claim 1 in the modification II which in the NIR spectrogram has peak maxima at 4086, 4228, 4418, 4457, 4634, 4905, 5846, 5911, 6026, 6081 and 6582 in combination with an inert, nontoxic, pharmaceutically suitable excipient.

8. Medicament comprising the compound of the formula (I) according to Claim 1 in the modification II which in the NIR spectrogram has peak maxima at 4086, 4228, 4418, 4457, 4634, 4905, 5846, 5911, 6026, 6081 and 6582 in combination with a further active substance.

9. Medicament according to Claim 7 or 8 for the treatment and/or prophylaxis of thromboembolic diseases.

10. Process for the prevention of blood coagulation in vitro, **characterized in that** an amount of the compound of the formula (I) according to Claim 1 having anticoagulatory activity in the modification II which in the NIR spectrogram has peak maxima at 4086, 4228, 4418, 4457, 4634, 4905, 5846, 5911, 6026, 6081 and 6582 is added.

## Revendications

1. Composé de formule (I) 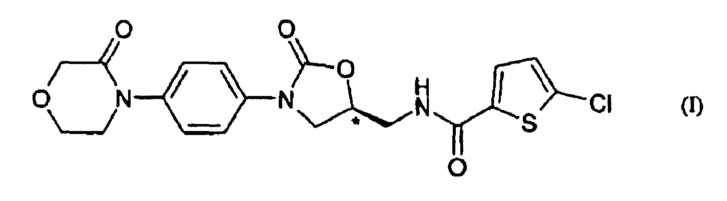 sous la forme II, qui présente sur le spectrogramme NIR des maximums de pic à 4 086, 4 228, 4 418, 4 457, 4 634, 4 905, 5 846, 5 911, 6 026, 6 081 et 6 582.

2. Procédé de fabrication du composé de formule (I) selon la revendication 1 sous la forme II, qui présente sur le spectrogramme NIR des maximums de pic à 4 086, 4 228, 4 418, 4 457, 4 634, 4 905, 5 846, 5 911, 6 026, 6 081 et 6 582, **caractérisé en ce que** le composé de formule (I) selon la revendication 1 sous la forme I, qui présente sur le spectrogramme NIR des maximums de pic à 4 082, 4 142, 4 170, 4 228, 4 299, 4 376, 4 429, 4 479, 4 633, 4 791, 4 877, 4 907, 5 081, 5 760, 5 885, 6 002, 6 441, 6 564, 8 473 et 8 833, est dissous dans du méthanol, de l'éthanol, du n-propanol, de l'iso-propanol, du n-butanol, du sec-butanol, de l'iso-butanol, du 1-pentanol ou de l'acétone ou du n-pentane, du cyclopentane, du n-hexane ou du tétrahydrofurane ou de l'acétonitrile ou de l'acétate d'éthyle ou du 1,4-dioxane ou un mélange des solvants cités ou un mélange des solvants cités avec de l'eau, et le composé est précipité par ajout de n-heptane, de cyclohexane ou de toluène.

3. Procédé de fabrication du composé de formule (I) selon la revendication 1 sous la forme II, qui présente sur le spectrogramme NIR des maximums de pic à 4 086, 4 228, 4 418, 4 457, 4 634, 4 905, 5 846, 5 911, 6 026, 6 081 et 6 582, **caractérisé en ce que** le composé de formule (I) selon la revendication 1 sous forme amorphe est suspendu dans du n-heptane, du cyclohexane ou du toluène, et la suspension est agitée ou secouée jusqu'à la transformation quantitative en la forme II.

4. Composé de formule (I) selon la revendication 1 sous la forme II, qui présente sur le spectrogramme NIR des maximums de pic à 4 086, 4 228, 4 418, 4 457, 4 634, 4 905, 5 846, 5 911, 6 026, 6 081 et 6 582, pour le traitement et/ou la prophylaxie de maladies.

5. Utilisation du composé de formule (I) selon la revendication 1 sous la forme II, qui présente sur le spectrogramme NIR des maximums de pic à 4 086, 4 228, 4 418, 4 457, 4 634, 4 905, 5 846, 5 911, 6 026, 6 081 et 6 582, pour la fabrication d'un médicament pour le traitement et/ou la prophylaxie de maladies thromboemboliques.

6. Utilisation du composé de formule (I) selon la revendication 1 sous la forme II, qui présente sur le spectrogramme NIR des maximums de pic à 4 086, 4 228, 4 418, 4 457, 4 634, 4 905, 5 846, 5 911, 6 026, 6 081 et 6 582, pour l'inhibition de la coagulation du sang in vitro.

7. Médicament contenant le composé de formule (I) selon la revendication 1 sous la forme II, qui présente sur le spectrogramme NIR des maximums de pic à 4 086, 4 228, 4 418, 4 457, 4 634, 4 905, 5 846, 5 911, 6 026, 6 081 et 6 582, en combinaison avec un adjuvant inerte, non toxique, pharmaceutiquement approprié.

8. Médicament contenant le composé de formule (I) selon la revendication 1 sous la forme II, qui présente sur le spectrogramme NIR des maximums de pic à 4 086, 4 228, 4 418, 4 457, 4 634, 4 905, 5 846, 5 911, 6 026, 6 081 et 6 582, en combinaison avec une autre substance active.

9. Médicament selon la revendication 7 ou 8 pour le traitement et/ou la prophylaxie de maladies thromboemboliques.

10. Procédé d'inhibition de la coagulation du sang in vitro, **caractérisé en ce qu'**une quantité anticoagulante efficace du composé de formule (I) selon la revendication 1 sous la forme II, qui présente sur le spectrogramme NIR des maximums de pic à 4 086, 4 228, 4 418, 4 457, 4 634, 4 905, 5 846, 5 911, 6 026, 6 081 et 6 582, est ajoutée.
